# EUROPEAN PATENT SPECIFICATION

(11) **EP 4 570 276 B1**
(45) Date of publication and mention of the grant of the patent: **31.12.2025**
(21) Application number: 24216329.3
(22) Date of filing: 29.11.2024
(51) Int. Cl.: A61L 27/16, A61L 27/18, A61L 27/34

(54) **A URINARY DRAINAGE PROSTHESIS FOR PATIENTS WITHOUT A BLADDER AND METHOD FOR MANUFACTURING IT**
HARNDRAINAGEPROTHESE FÜR BLASENLOSE PATIENTEN UND VERFAHREN ZU IHRER HERSTELLUNG
PROTHÈSE DE DRAINAGE URINAIRE POUR PATIENTS SANS VESSIE ET SON PROCÉDÉ DE FABRICATION

(30) Priority: 30.11.2023 PL 44693623
(43) Date of publication of application: 18.06.2025
(73) Proprietor: Uniwersytet Mikolaja Kopernika w Toruniu, 87-100 Torun (PL); Kwiecinski, Piotr, 62-720 Brudzew (PL); Siec Badawcza Lukasiewicz Lodzki Instytut Technologiczny, 90-570 Lodz (PL); Instytut Podstawowych Problemów Techniki Polskiej Akademii Nauk, 02-106 Warszawa (PL); Meger, Katarzyna, 85-737 Bydgoszcz (PL)
(72) Inventor: Kwiecinski, Piotr, 62-720 Brudzew (PL); Meger, Katarzyna, 85-737 Bydgoszcz (PL); Adamiec, Wieslaw, 91-496 Lodz (PL); Drewa, Tomasz, 85-066 Bydgoszcz (PL); Jundzill, Arkadiusz, 85-363 Bydgoszcz (PL); Kowalczyk, Tomasz, 05-119 Legionowo (PL); Kucharska, Magdalena, 94-056 Lodz (PL); Meger, Marian, 85-724 Bydgoszcz (PL); Niemczyk-Soczynska, Beata, 37-300 Lezajsk (PL); Pokrywczynska, Marta, 88-180 Zlotniki Kujawskie (PL); Tomaszewski, Waclaw, 95-100 Zgierz (PL); Urbanek-Swiderska, Olga, 05-092 Lomianki (PL); Zembrzycki, Krzysztof, 05-825 Grodzisk Mazowiecki (PL)
(74) Representative: Czarnik, Maciej

(56) References cited:
- WO-A1-2010/043177
- CN-A- 116 536 246
- US-A1- 2008 038 352
- US-A1- 2016 262 865
- US-A1- 2018 243 482

## Description

The subject of the invention is the prosthesis for urinary drainage for patients following radical cystectomy and the method of manufacturing it. The invention finds application in medicine, in patients following radical cystectomy being the consequence of a disease, particularly a cancer disease, or due to urinary bladder trauma and when it can no longer work properly.

The solutions concerning the reconstruction of the lower urinary tract are already known. The French patent FR2116838 reveals an artificial urinary bladder connected to two ureters and the urethra of a patient and it is equipped with three internal flap valves, two of which enable the inflow from the ureters and at the same time, the third one operates reversely to the two flap valves (drain). Moreover, the artificial urinary bladder is equipped with a flap control device, which transmits control via gas in a separate system. Additionally, the system includes a device for controlling pressure in the urinary bladder. CN116536246A discloses a tubular artificial urethra comprising at least a loose and a dense layer, which can be produced by electrospinning polymers such as PGA, PCL, PET, PP and collagen.

In solutions involving the use of the artificial urinary bladder, a connection between the natural ureters, urethra, and the artificial system is problematic. When the human tissue with its blood supply is connected to artificial systems, insufficient tissue healing may occur, which results in local leakages. Moreover, the defensive reactions of the human body against artificial systems constitute a potential danger to a patient's life. Peritonitis with multi-organ disorders within the abdominal cavity is a possible consequence. Surgical interventions undertaken to eliminate the disorders are, *per se,* serious and risky procedures.

Alternative methods of urine drainage after radical cystectomy based on the implantation of urine transporting drains through the connection with an extracorporeal urine collection and removal system which allows its storage (e.g., a percutaneous nephrostomy or ureterostomy with a stoma bag) are also possible. Although such methods cause a specific reduction in quality and comfort of life, the procedures are much safer and have fewer complications.

The fundamental problem concerning the given invention is to ensure the best possible biocompatibility of the prosthesis with a patient's body.

According to the invention, the urinary drainage prosthesis is composed of a polypropylene or polyester mesh tube modified with the use of cold oxygen plasma to increase its hydrophilicity, with a mat in the form of a cone-shaped collar attached to the distal end, produced by electrospinning of a polyester solution, with the modified surface to increase hydrophilicity, whereby the internal side of the tube is coated with a layer of cross-linked sodium hyaluronate in the form of a film and the external side of the tube is coated with a nanofiber layer made of cross-linked collagen, on which the mesenchymal stem cells are seeded in a preferred version of the invention.

The subject of the invention is therefore a prosthesis for urinary drainage for patients following radical cystectomy characterized in that it contains a tube which is made of a hydrophilic matrix made of polypropylene or polyester mesh, ended with a collar on one side, preferably in a cone-shaped form, made of micro and/or nanofiber nonwoven fabric prepared from a synthetic fiber-forming polymer or copolymers, natural polymers such as esters, cellulose ethers, collagen, gelatin, fibrinogen, natural silk; coated on the internal surface with a layer of cross-linked sodium hyaluronate of the average molecular weight from 2 000 kD to 2 500 kD in the form of a film with an addition of the plasticizer - a polyol, preferably glycerin in the amount from 10% w/w to 20% w/w of dry sodium hyaluronate, and on the external surface with a layer of type I collagen of the animal origin of the average molecular weight from 250 kD to 350 kD in the form of a nanofiber layer, whereby the sodium hyaluronate layer amounts from 15% w/w to 25% w/w, preferably 20% w/w of the mass of the prosthesis, and the collagen layer - from 20% w/w to 40% w/w, preferably 30% w/w of the mass of the prosthesis.

Preferably, the tube is from 8 mm to 12 mm in diameter and 50 mm to 100 mm in length.

Preferably, the size of the polypropylene mesh or polyester mesh in the hydrophilic matrix amounts from 35 µm to 5100 µm, preferably from 250 µm to 350 µm and the spun fiber thickness from 90 µm to 1000 µm, optimally from 150 µm to 350 µm.

Preferably the collar is made of micro and/or nanofiber nonwoven fabric obtained from a synthetic fiber-forming polymer soluble in organic solvents such as polylactide, polyglycolide, polycaprolactone, polyamide, polyimide, poly(trimethylene carbonate), polydioxanone, polyvinyl acetate, polyvinyl alcohol, poly(N-vinyl-2-pyrrolidone), polyvinyl butyral, polyurethane, poly(ether urethane), polycarbonate, aromatic polycarbonate, poly(ethylene oxide), poly(ethylene glycol), poly(methyl methacrylate), poly(acrylic acid), polyacrylamide, polyacrylonitrile, polyvinyl chloride, poly(vinylidene fluoride), poly(tetrafluoroethylene), poly(ethylene glycol terephthalate), or their mixtures.

Preferably, the collar is made of a micro and/or nanofiber nonwoven fabric obtained from a cellulose ester chosen from the group: carboxymethylcellulose, carboxymethylcellulose, methylcellulose, or their mixtures.

Preferably, the collar is made of a micro and/or nanofiber nonwoven fabric obtained from a copolymer chosen from the group: L-lactide and caprolactone copolymer, poly(L-lactide-co-caprolactone), or their mixtures.

Preferably, the external surface of the tube is coated with autologous or allogenic mesenchymal stem/ stromal cells isolated from bone marrow, adipose tissue, or Wharton's jelly.

The subject of the method of manufacturing a prosthesis for urinary drainage for patients with planned radical cystectomy is characterized in that the external and internal surfaces of a hydrophilic matrix made of polypropylene or polyester mesh of a tubular shape are modified with cold oxygen plasma to increase their hydrophilicity. Next, under dynamic conditions, using the (spraying and coating or hard-facing and coating) method, the internal surface is coated with layers of an aqueous solution of sodium hyaluronate at a concentration from 0.7% w/w to 1.2% w/w, preferably 0.7% w/w crosslinked with the use of butanediol-1,4-ol diglycidyl ether (BDDE) in the amount from 10% w/w to 15% w/w (in terms of dry polymer content) with the addition of a plasticizer in the amount from 10% w/w to 20% w/w (in terms of dry polymer content), whereby each of the consecutively deposited layers of crosslinked sodium hyaluronate undergoes the drying process at the temperature from 30°C to 40°C. After all the layers of crosslinked sodium hyaluronate are deposited, the prosthesis undergoes the rinsing process in an aqueous solution of ethyl alcohol at the concentration of 70% w/w and 30% w/w and rinsing twice in demineralized water with the addition of a plasticizer in the amount of 5% w/w, and next freeze drying in the vacuum range from 0.1 mbar to 0.57 mbar for 20-22 hours. Next, on the external surface of the prosthesis, a nanofiber layer is deposited, which is produced with the use of the electrospinning and/or electrospraying method from a collagen solution of animal origin, dissolved in hexafluoroisopropanol (HFIP) at the concentration from 0.1% w/w to 0.3% w/w, preferably 0.3% w/w. After coating with the collagen layer, the prosthesis undergoes the crosslinking process in the alcohol solution of N-(3-dimethylaminopropyl)-N'-ethylcarbodiimide (EDC) at the concentration from 5.0% w/w to 5.5% w/w, preferably 5% w/w, for 5-7 days, and next the rinsing process in aqueous solutions of ethyl alcohol 70% w/w and 30% w/w and demineralized water with the addition of a plasticizer in the amount of 5% w/w, and the freeze drying in the vacuum range from 0.1 mbar to 0.57 mbar for 20-22 hours.

Preferably in the method according to the invention, the external surface of the prosthesis for urinal drainage is coated with autologous or allogenic mesenchymal phalangeal/ mesenchymal stem/stromal cells isolated from bone marrow, adipose tissue, or Wharton's jelly seeded at the density of 1-100 x 10⁶ cells/cm², preferably 10 x 10⁶ cells/ cm², whereby the cells are cultivated on the prosthesis for the period of 1-30 days, preferably 7 days.

The research on a large animal model showed a significant impact of using mesenchymal stem/ stromal cells on the healing processes in reconstructions using the prosthesis in the urinary tract.

The table below compares the inflammatory reactions in the mesh coated with collagen and the mesh coated with collagen seeded with adipose-derived mesenchymal stem/stromal cells one month after the prosthesis implantation, according to the invention.

| | Average time of patency of urinary fistula (months) | Average time of the insert prolapse (months) |
|---|---|---|
| Control group - ureterocutaneostomia | 6 | 0 |
| Reconstruction of the ureter with the prosthesis according to the invention, with the use of tissue glue | 12 | 5 |
| Reconstruction of the ureter with the pre-implanted prosthesis according to the invention, seeded with mesenchymal stem/stromal cells and with the use of tissue glue | 18 | 8 |

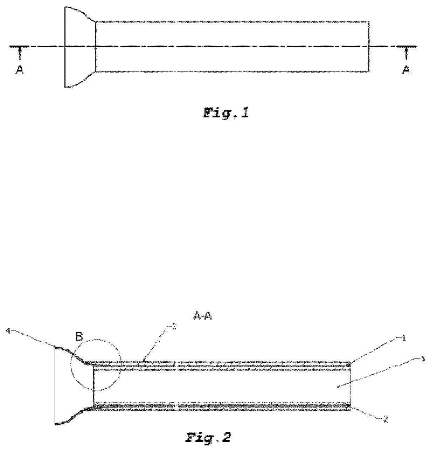

The invention has been presented in embodiments, and the Scheme in which Fig. 1 shows a schematic view of the prosthesis construction, Fig. 2 shows the section of the prosthesis construction.

### Example 1

A rectangle of polypropylene mesh is modified with cold oxygen plasma to increase the hydrophilicity of the surface and then welded to form a tube of 10 mm in diameter. A previously prepared solution containing 9 w/w of poly(L-lactide-co-caprolactone), 85 w/w chloroform, and 6 w/w N, N-dimethylformamide is subject to the electrospinning process under the following conditions: the nozzle-collector distance - 20 cm, the temperature 23°C, the nozzle collector voltage - 15kV. The electrospun nonwoven fabric is collected on a grounded rotating cylinder of the tubular shape ending with the cone generatrix. The obtained mat is placed in a 10% sodium bicarbonate solution NaHCO₃ for 40 days to increase the surface hydrophilicity. After rinsing and drying, the collar, prepared with a micro and nanofiber nonwoven fabric tube, is sewed to the previously prepared polypropylene mesh tube, which was hydrophilically modified.

Next, under dynamic conditions, using spraying and deposition, the internal surface is coated with a layer of an aqueous solution of sodium hyaluronate at the concentration of 0.7% w/w crosslinked with the use of butanediol-1,4-ol diglycidyl ether (BDDE) in the amount of 15% w/w (in terms of dry polymer content) with the addition of a plasticizer in the amount from 10% w/w (in terms of dry polymer content), whereby each of the consecutively deposited layers of crosslinked sodium hyaluronate undergoes the drying process at the temperature of 35°C. After all the layers of crosslinked sodium hyaluronate are deposited, the prosthesis undergoes the rinsing process in an aqueous solution of ethyl alcohol at the concentration of 70% w/w and 30% w/w and rinsing twice in demineralized water with the addition of a plasticizer in the amount of 5% w/w, and next freeze drying in the vacuum range from 0.1 mbar to 0.57 mbar for 20-22 hours. Next, on the external surface of the prosthesis, a nanofiber layer is deposited, which is produced with the use of the electrospinning method from a collagen solution of animal origin, dissolved in hexafluoroisopropanol (HFIP) at the concentration of 0.3% w/w. After coating with the collagen layer, the prosthesis undergoes the crosslinking process in the alcohol solution of N-(3-dimethylaminopropyl)-N'-ethylcarbodiimide (EDC) at the concentration of 5.0% w/w for 7 days and following the rinsing process in aqueous solutions of ethyl alcohol 70% w/w and 30% w/w and demineralized water with the addition of a plasticizer in the amount of 5% w/w, and the freeze drying in the vacuum range from 0.1 mbar to 0.57 mbar for 20-22 hours.

### Example 2.

To increase their hydrophilicity, a previously welded polypropylene mesh tube is modified with cold oxygen plasma. A previously prepared solution containing 9 w/w of poly(L-lactide-co-caprolactone), 85 w/w chloroform, and 6 w/w N, N-dimethylformamide is subject to the electrospinning process under the following conditions: the nozzle-collector distance - 20 cm, the temperature 23°C, the nozzle collector voltage - 15kV. The thus obtained nonwoven fabric undergoes hydrophilic modification by immersion in the 8% solution of potassium carbonate K₂CO₃ for 48 hours. After rinsing and drying, the prepared collar is sewn to the previously prepared polypropylene mesh tube.

Next, under dynamic conditions, using spraying and deposition, the internal surface is coated with a layer of an aqueous solution of sodium hyaluronate at the concentration of 1.2% w/w crosslinked with the use of butanediol-1,4-ol diglycidyl ether (BDDE) in the amount of 15% w/w (in terms of dry polymer content) with the addition of a plasticizer in the amount of 20% w/w (in terms of dry polymer content), whereby each of the consecutively deposited layers of crosslinked sodium hyaluronate undergoes the drying process at the temperature of 35°C. After all the layers of crosslinked sodium hyaluronate are deposited, the prosthesis undergoes the rinsing process in an aqueous solution of ethyl alcohol at the concentration of 70% w/w and 30% w/w and rinsing twice in demineralized water with the addition of a plasticizer in the amount of 5% w/w, and next freeze drying in the vacuum range from 0.1 mbar to 0.57 mbar for 20-22 hours.

Next, on the external surface of the prosthesis, a nanofiber layer is deposited, which is produced with the use of the electrospinning and electrospraying method from a collagen solution of animal origin, dissolved in hexafluoroisopropanol (HFIP) at the concentration of 0.1% w/w. After coating with the collagen layer, the prosthesis undergoes the crosslinking process in the alcohol solution of N-(3-dimethylaminopropyl)-N`-ethylcarbodiimide (EDC) at the concentration of 5.5% w/w for 5 days and following the rinsing process in aqueous solutions of ethyl alcohol 70% w/w and 30% w/w and demineralized water with the addition of a plasticizer in the amount of 5% w/w, and the freeze drying in the vacuum range from 0.1 mbar to 0.57 mbar for 20-22 hours.

### Example 3.

The prosthesis for urinary drainage schematically presented in Fig. 2 comprises matrix 1 in the tubular shape made of polypropylene mesh. The internal side of matrix 2 is coated with a film formed by crosslinked hyaluronic acid with an average molecular weight of 1320 kD, with glycerin as the plasticizer of 20% w/w. The external surface 3 of the polypropylene matrix 1 is coated with an active nanofiber layer of type 1 collagen of animal origin of an average molecular weight of 300 kD. The distal end is equipped with a collar 4 prepared of poly(L-lactide-co-caprolactone) in the nanofiber nonwoven, enabling a 10 mm clearance hole 5 in the prosthesis.

The advantage of the invention over known solutions is the possibility of preparing the prosthesis outside a patient's organism, which allows the accurate adjustment of its parameters to the predicted biochemical model of the artificial urine outlet functioning in a given patient, as well as prosthesis adjustment to a patient's variability, and hence, the patient's prognoses and quality of life in future.

The superficially modified prosthesis is characterized by the permanent adhesion of polymer layers to the synthetic matrix, complete tightness preventing urine leakage, the developed internal surface enabling the settlement of the cells initiating the growth of the proper natural tissue, and good integration with animal tissue. Applying the prosthesis according to the invention allows for significant shortening of the urinary diversion procedure following radical cystectomy about the known methods.

## Claims

1. A urinary drainage prosthesis for patients following radical cystectomy is **characterized in that** it comprises a tube made of a hydrophilic matrix made of polypropylene or polyester mesh in the shape of a tube, ended with a collar on one side, preferably in a cone-shaped form, made of micro and/or nanofiber nonwoven fabric prepared from a synthetic fiber-forming polymer or copolymers, natural polymers such as esters, cellulose ethers, collagen, gelatin, fibrinogen, natural silk; coated on the internal surface with a layer of cross-linked sodium hyaluronate of the average molecular weight from 2 000 kD to 2 500 kD in the form of a film with an addition of the plasticizer - a polyol, preferably glycerin in the amount from 10% w/w to 20% w/w of dry sodium hyaluronate, and on the external surface with a layer of type I collagen of the animal origin of the average molecular weight from 250 kD to 350 kD in the form of a nanofibrilar layer, whereby the sodium hyaluronate layer amounts from 15% w/w to 25% w/w, preferably 20% w/w in terms of the mass of the prosthesis, and the collagen layer - from 20% w/w to 40% w/w, preferably 30% w/w in terms of the mass of the prosthesis.

2. The urinary drainage prosthesis according to claim 1, **characterized in that** the tube is from 8 mm to 12 mm in diameter and 50 mm to 100 mm in length.

3. The urinary drainage prosthesis according to claim 1, **characterized in that** the size of the polypropylene mesh or polyester mesh in the hydrophilic matrix amounts from 35 µm to 5100 µm, preferably from 250 µm to 350 µm and the spun fiber thickness from 90 µm to 1000 µm, optimally from 150 µm to 350 µm.

4. The urinary drainage prosthesis according to claim 1, **characterized in that** the collar is made of micro and/or nanofiber nonwoven fabric obtained from a synthetic fiber-forming polymer soluble in organic solvents such as polylactide, polyglycolide, polycaprolactone, polyamide, polyimide, poly(trimethylene carbonate), polydioxanone, polyvinyl acetate, polyvinyl alcohol, poly(N-vinyl-2-pyrrolidone), polyvinyl butyral, polyurethane, poly(ether urethane), polycarbonate, aromatic polycarbonate, poly(ethylene oxide), poly(ethylene glycol), poly(methyl methacrylate), poly(acrylic acid), polyacrylamide, polyacrylonitrile, polyvinyl chloride, poly(vinylidene fluoride), poly(tetrafluoroethylene), poly(ethylene glycol terephthalate), or their mixtures.

5. The urinary drainage prosthesis according to claim 1, **characterized in that** the collar is made of a micro and/or nanofiber nonwoven fabric produced of a cellulose ester chosen from the group: carboxymethylcellulose carboxymethylcellulose, methylcellulose, or their mixtures.

6. The urinary drainage prosthesis according to claim 1, **characterized in that** the collar is made of a micro and/or nanofiber nonwoven fabric obtained from a copolymer chosen from the group: L-lactide and caprolactone copolymer, poly(L-lactide-co-caprolactone), or their mixtures.

7. The urinary drainage prosthesis according to any one of claims 1-6, **characterized in that** the external surface of the tube is coated with autologous or allogenic mesenchymal stem/stromal cells isolated from bone marrow, adipose tissue, or Wharton's jelly.

8. A method of manufacturing the urinary drainage prosthesis for patients following radical cystectomy **characterized in that** the external and internal surfaces of a hydrophilic matrix made of polypropylene or polyester mesh of a tubular shape are modified with cold oxygen plasma in order to increase their hydrophilicity and next, using the spraying and coating, or hardfacing and coating method, the internal surface is coated with layers of an aqueous solution of sodium hyaluronate at a concentration from 0.7% w/w to 1.2% w/w, preferably 0.7% w/w with the addition of a plasticizer in the amount from 10% w/w to 20% w/w (in terms of dry polymer content), crosslinked with the use of butanedi-1,4-ol diglycidyl ether (BDDE) in the amount from 10% w/w to 15% w/w in terms of polymer content, whereby each of the consecutively deposited layers of crosslinked sodium hyaluronate undergoes the drying process at the temperature of 35°C, and after all the layers of crosslinked sodium hyaluronate are deposited, the prosthesis undergoes the rinsing process in an aqueous solution of ethyl alcohol at the concentration of 70% w/w and 30% w/w, and rinsing twice in demineralized water with the addition of a plasticizer in the amount of 5% w/w, and next freeze drying in the vacuum range from 0.1 mbar to 0.57 mbar for 20-22 hours and next, on the external surface of the prosthesis, a nanofiber layer is deposited which is produced with the use of the electrospinning and/or electrospraying method from a solution of collagen of the animal origin, dissolved in hexafluoroisopropanol (HFIP) at the concentration from 0.1% w/w to 0.3% w/w, preferably 0.3% w/w, and after coating with the collagen layer, the prosthesis undergoes the crosslinking process in the alcohol solution of N-(3-dimethylaminopropyl)-N'-ethylcarbodiimide (EDC) at the concentration from 5.0% w/w to 5.5% w/w, preferably 5% w/w, for 5-7 days, and next the rinsing process in aqueous solutions of ethyl alcohol 70% w/w and 30% w/w and demineralized water with the addition of a plasticizer in the amount of 5% w/w, and the freeze drying in the vacuum range from 0.1 mbar to 0.57 mbar for 20-22 hours.

9. The method of manufacturing the prosthesis according to claim 8, **characterized in that** the external surface of the prosthesis is seeded with autologous or allogenic mesenchymal stem/stromal cells isolated from bone marrow, adipose tissue, or Wharton's jelly at the density from 1 to 100 x 10⁶ cells/cm², preferably 10 x 10⁶ cells/cm², and the cells cultivation on the surface of prosthesis is performed under static or dynamic conditions, preferably in a bioreactor for 1-30 days, preferably 7 days.

## Patentansprüche

1. Prothese zur Ableitung von Harn für Patienten, bei denen die Harnblase entfernt wurde, **dadurch gekennzeichnet, dass** sie einen Schlauch enthält, der eine hydrophile Matrix aus Polypropylen- oder Polyesternetz in Schlauchform enthält, abgeschlossen von einer Seite mit einem Flansch, vorzugsweise in Form eines Kegels, ausgeführt aus einer Faser aus Mikro- und/oder Nanofasern, die aus synthetischem, faserbildendem Polymer gewonnen wurden, oder Copolymeren, natürlichen Polymeren, wie: Celluloseester und -ether, Kollagen, Gelatine, Fibrinogen, natürlicher Seide; beschichtet auf einer Seite mit einer Schicht aus vernetztem Natriumhyaluronat mit einem durchschnittlichen Molekulargewicht von 2 000 kD bis 2 500 kD in Form eines Films mit Zusatz eines Fließmittels - Polyalkohol, vorzugsweise Glycerin in einer Menge von 10 Gew.-% bis 20 Gew.-%, im Verhältnis zum trockenen Natriumhyaluronat, und auf der Außenfläche mit einer Schicht Kollagen Typ I tierischer Herkunft, mit einem durchschnittlichen Molekulargewicht zwischen 250 kD und 350 kD, in Form einer Nanofaserschicht, wobei die Natriumhyaluronatschicht zwischen 15 Gew.-% und 25 Gew.-%, vorzugsweise 20 Gew.-%, im Verhältnis zur Masse der Prothese, und die Kollagenschicht zwischen 20 Gew.-% und 40 Gew.-%, vorzugsweise 30 Gew.-% beträgt, im Verhältnis zur Masse der Prothese.

2. Prothese zur Ableitung von Harn gemäß Anspruch 1, **dadurch gekennzeichnet, dass** der Schlauch einen Durchmesser zwischen 8 mm und 12 mm und eine Länge zwischen 50 mm und 100 mm hat.

3. Prothese zur Ableitung von Harn gemäß Anspruch 1, **dadurch gekennzeichnet, dass** die Öffnungsgröße des Polypropylennetzes oder der hydrophilen Polyestermatrix zwischen 35 µm und 5 100 µm, vorzugsweise 250 µm bis 350 µm beträgt, mit einer Garndicke zwischen 90 µm und 1 000 µm, optimal zwischen 150 µm und 350 µm.

4. Prothese zur Ableitung von Harn gemäß Anspruch 1, **dadurch gekennzeichnet, dass** der Flansch aus einer Faser aus Mikro- und/oder Nanofasern hergestellt ist, die aus synthetischem, faserbildendem Polymer gewonnen wurden, das ein in organischen Lösungsmitteln lösbares Polymer ist - wie Polylactid, Polyglycolid, Poly(caprolacton), Polyamid, Polyimid, Poly(trimethylencarbonat), Poly(dioxanon), Poly(vinylacetat), Poly(vinylalkohol), Poly(N-vinyl-2-pyrrolidon), Poly(vinylbutyral), Polyurethan, Poly(etherurethan), Polycarbonat, aromatisches Polycarbonat, Poly(ethylenoxid), Poly(ethylenglykol), Poly(methylacrylat), Poly(methylmethacrylat), Poly(acrylsäure), Poly(acrylamid), Poly(acrylnitril), Poly(vinylchlorid), Poly(vinylidenfluorid), Poly(tetrafluorethylen), Polyethylenglykol-Terephthalat oder deren Gemische.

5. Prothese zur Ableitung von Harn gemäß Anspruch 1, **dadurch gekennzeichnet, dass** der Flansch aus einer Faser aus Mikro- und/oder Nanofasern hergestellt ist, die aus einem Cellulaseester gewonnen wurden, der aus der Gruppe ausgewählt wurde: Carboxymethylcellulose, Carboxyethylcellulose, Methylcellulose oder deren Gemische.

6. Prothese zur Ableitung von Harn gemäß Anspruch 1, **dadurch gekennzeichnet, dass** der Flansch aus einer Faser aus Mikro- und/oder Nanofasern hergestellt ist, die aus einem Copolymer gewonnen wurden, das aus der Gruppe ausgewählt wurde: **L-**Lactid- oder Caprolacton-Polymer, Poly(L-Lactid-Cocaprolacton), oder deren Gemische.

7. Prothese zur Ableitung von Harn nach einem der Ansprüche 1 - 6, **dadurch gekennzeichnet, dass** die äußere Fläche des Schlauchs mit autologen oder allogenen mesenchymalen Stromazellen/Stammzellen beschichtet ist, die aus dem Knochenmark, dem Fettgewebe oder der Wharton-Sulze gewonnen wurden.

8. Herstellungsverfahren der Prothese zur Ableitung von Harn für Patienten, bei denen die Harnblase entfernt wurde, **dadurch gekennzeichnet, dass** die äußere und innere Oberfläche der aus einem Polypropylen- oder Polyesternetz hergestellten Matrix in Form eines Schlauchs mit kaltem Sauerstoffplasma modifiziert wird, um die Hydrophilie zu steigern, und anschließend auf die innere Oberfläche, unter Anwendung eines Sprüh- und Beschichtungsverfahrens oder eines Auftrag- und Beschichtungsverfahrens eine Schicht wässriger Natriumhyaluronatlösung aufgetragen wird, mit einer Konzentration zwischen 0,7 Gew.-% und 1,2 Gew.-%, vorzugsweise 0,7 Gew.-%, mit Zusatz eines Fließmittels in einer Menge zwischen 10 Gew.-% und 20 Gew.-% (umgerechnet auf den Gehalt an trockenem Polymer), vernetzt mithilfe von Butandiol-1,4-diglycidylether (BDDE) in einer Menge zwischen 10 Gew.-% und 15 Gew.-%, umgerechnet auf den Polymergehalt, wobei jede der nacheinander aufgetragenen Schichten des vernetzten Natriumhyaluronats einem Prozess der Trocknung bei einer Temperatur von 35 °C unterzogen wird, und nach dem Auftragen aller Schichten des vernetzten Natriumhyaluronats die Prothese dem Prozess der Spülung unterzogen wird, in einer wässrigen Ethyl-Ethanol-Lösung mit einer Konzentration von 70 Gew.-% und 30 Gew.-%, sowie der zweifachen Spülung in demineralisiertem Wasser mit dem Zusatz eines Fließmittels in einer Menge von 0,5 Gew.-%, und anschließend einer Sublimationstrocknung im Vakuumbereich von 0,1 mbar bis 0,57 mbar für einen Zeitraum von 20-22 Stunden, wobei auf der äußeren Oberfläche der Prothese eine Nanofaserschicht aufgetragen wird, die mit dem Verfahren des Elektrospinnens oder des Elektrospinnens und des Elektrosprays hergestellt wurde, aus einer Kollagenlösung tierischer Herkunft, aufgelöst in Hexafluorisopropanol (HFIP) mit einer Konzentration von 0,1 Gew.-% bis 0,3 Gew.-%, vorzugsweise 0,3 Gew.-%, und nach dem Auftragen der Kollagenschicht, die Prothese dem Prozess der Vernetzung unterzogen wird, in einer alkoholischen Lösung N-[3-(Dimethylamin)Propyl]-N'-Ethylcarbodiimid (EDE) mit einer Konzentration zwischen 5,0 Gew.-% bis 5,5 Gew.-%, vorzugsweise 5 Gew.-%, während einer Zeit von 5-7 Tagen, und anschließend einem Prozess der Spülung in wässrigen Lösungen von Ethylalkohol 70 Gew.-% und 30 Gew.-% und in demineralisiertem Wasser mit dem Zusatz eines Fließmittels in einer Menge von 5 Gew.-% sowie dem Prozess der Sublimationstrocknung im Vakuumbereich von 0,1 mbar bis 0,57 mbar für einen Zeitraum von 20-22 Stunden.

9. Herstellungsverfahren der Prothese gemäß Anspruch 8, **dadurch gekennzeichnet, dass** die äußere Oberfläche der Prothese mit autologen oder allogenen mesenchymalen Stromazellen/Stammzellen ausgekleidet wird, die aus dem Knochenmark, dem Fettgewebe oder der Wharton-Sulze gewonnen wurden, mit einer Dichte von 1 bis 100 x 10⁶ Zellen/cm², vorzugsweise 10⁶ Zellen/cm², und die Zellkultur auf der Oberfläche der Prothese unter statischen oder dynamischen Bedingungen geführt wird, vorzugsweise in einem Bioreaktor für einen Zeitraum von 1-30 Tagen, vorzugsweise 7 Tagen.

## Revendications

1. Prothèse de dérivation urinaire pour patients ayant subi une ablation de la vessie, **caractérisée en ce qu'**elle comprend un tube, qui est une matrice hydrophile en maille de polypropylène ou de polyester en forme de tube, terminée d'un côté par une collerette, de préférence en forme de cône, en micro et/ou nanofibres non tissées obtenues à partir d'un polymère synthétique formant des fibres, ou de copolymères, de polymères naturels tels que : esters et éthers de cellulose, collagène, gélatine, fibrinogène, soie naturelle ; recouverte sur la face interne d'une couche de hyaluronate de sodium réticulé d'un poids moléculaire moyen de 2 000 kD à 2 500 kD, sous forme de film avec l'ajout d'un plastifiant : alcool polyhydrique, de préférence la glycérine, dans une quantité de 10 % en poids à 20 % en poids, par rapport au poids du hyaluronate de sodium sec, et sur la surface externe, d'une couche de collagène de type I d'origine animale d'un poids moléculaire moyen de 250 kD à 350 kD sous forme d'une couche nanofibreuse, la couche de hyaluronate de sodium étant de 15 % en poids à 25 % en poids, de préférence 20 % en poids par rapport au poids de la prothèse, et la couche de collagène étant de 20 % en poids à 40 % en poids, de préférence 30 % en poids par rapport au poids de la prothèse.

2. Prothèse de dérivation urinaire selon la revendication 1, **caractérisée en ce que** le tube a un diamètre de 8 mm à 12 mm et une longueur de 50 mm à 100 mm.

3. Prothèse de dérivation urinaire selon la revendication 1 **caractérisé en ce que** la taille des mailles en polypropylène ou en polyester hydrophile est compris entre 35 µm et 5100 µm, de préférence entre 250 µm et 350 µm, et que l'épaisseur du fil est comprise entre 90 µm et 1000 µm, de préférence entre 150 µm et 350 µm.

4. Prothèse de dérivation urinaire selon la revendication 1, **caractérisé en ce que** la collerette est constituée de micro et/ou nanofibres non tissées obtenues à partir d'un polymère synthétique formant des fibres qui est un polymère soluble dans les solvants organiques, tel que : poly(lactide), poly(glycolide), poly(caprolactone), polyamide, polyimide, poly(carbonate de triméthylène), poly(dioxanone), poly(alcool vinylique), poly(N-vinyl-2-pyrrolidone), poly(butyral vinylique), polyuréthane, poly(éther uréthane), polycarbonate aromatique, poly(oxyde d'éthylène), poly(éthylène glycol), poly(acrylate de méthyle), poly(méthacrylate de méthyle), poly(acide acrylique), poly(acrylamide), poly(acrylonitrile), poly(chlorure de vinyle), poly(fluorure de vinylidène), poly(tétrafluoroéthylène), poly(téréphtalate d'éthylène glycol), un mélange de ceux-ci.

5. Prothèse de dérivation urinaire selon la revendication1, **caractérisée en ce que** la collerette est constituée de micro et/ou nanofibres non tissées obtenues à partir d'un ester de cellulase choisi dans le groupe constitué par : la carboxyméthylcellulose, la carboxyéthylcellulose, la méthylcellulose, ou un mélange de ceux-ci.

6. Prothèse de dérivation urinaire selon la revendication 1, **caractérisée en ce que** la collerette est constituée de micro et/ou nanofibres non tissées obtenues à partir d'un copolymère choisi dans le groupe constitué par : copolymère de L-lactide et de caprolactone, poly(L-lactide-co-caprolactone), ou un mélange de ceux-ci.

7. Prothèse de dérivation urinaire selon l'une quelconque des revendications 1 à 6, **caractérisée en ce que** la surface externe du tube est recouverte de cellules mésenchymateuses stromales/souches autologues ou allogéniques isolées de la moelle osseuse, du tissu adipeux ou de la gelée de Wharton.

8. Procédé de fabrication d'une prothèse de dérivation urinaire pour patients ayant subi une ablation de la vessie, **caractérisé en ce que** la surface externe et interne de la matrice réalisée à partir d'une maille en polypropylène ou en polyester, de forme tubulaire, est modifiée par plasma froid d'oxygène afin d'augmenter son pouvoir hydrophile ; puis on applique sur la surface interne, au moyen d'un procédé de pulvérisation par projection ou de dépôt et revêtement, une couche d'une solution aqueuse d'hyaluronate de sodium à une concentration comprise entre 0,7 % en poids et 12 % en poids, de préférence 0,7 % en poids, contenant un plastifiant en une quantité comprise entre 10 % en poids et 20 % en poids (exprimée par rapport à la masse du polymère sec), et réticulée au moyen de l'éther diglycidylique de 1,4-butanediol (BDDE) en une quantité comprise entre 10 % en poids et 15 % en poids par rapport au poids du polymère ; chaque couche successivement appliquée d'hyaluronate de sodium réticulé étant soumise à un procédé de séchage à 35 °C ; et après l'application de l'ensemble des couches d'hyaluronate de sodium réticulé, la prothèse étant soumise à un procédé de rinçage dans une solution aqueuse d'éthanol éthylique à 70 % en poids puis 30 % en poids, ainsi qu'à un rinçage double dans de l'eau déminéralisée contenant 5 % en poids de plastifiant, suivi d'un séchage par sublimation sous une pression comprise entre 0,1 mbar et 0,57 mbar pendant 20 à 22 heures ; après quoi une couche nanofibreuse est déposée sur la surface externe de la prothèse, ladite couche étant obtenue par électrofilage ou par électrofilage et électro-spray à partir d'une solution de collagène d'origine animale dissous dans l'hexafluoroisopropanol (HFIP) à une concentration comprise entre 0,1 % en poids et 0,53 % en poids, de préférence 0,53 % en poids ; et après l'application de la couche de collagène, la prothèse est soumise à un procédé de réticulation dans une solution alcoolique de N-(p-(diméthylamino)propyl)-N'-éthylcarbodiimide (EDE) à une concentration comprise entre 0,50 % en poids et 5,5 % en poids, de préférence 5 % en poids, pendant 5 à 7 jours ; puis à un procédé de rinçage dans des solutions aqueuses d'éthanol à 70 % en poids et 30 % en poids, ainsi que dans de l'eau déminéralisée contenant 5 % en poids de plastifiant, et enfin à un séchage par sublimation sous une dépression comprise entre 0,51 mbar et 0,557 mbar pendant 20 à 22 heures.

9. Procédé de fabrication de la prothèse selon la revendication 8, **caractérisé en ce que** la surface externe de la prothèse est ensemencée avec des cellules mésenchymateuses stromales/souches autologues ou allogéniques, isolées de la moelle osseuse, du tissu adipeux ou de la gelée de Wharton, à une densité comprise entre 1 et 100 × 10⁶ cellules/cm², de préférence 10 × 10⁶ cellules/cm², et la culture des cellules sur la surface de la prothèse est conduite dans des conditions statiques ou dynamiques, de préférence dans un bioréacteur, pendant une période de 1 à 30 jours, de préférence 7 jours.
